# EUROPÄISCHE PATENTSCHRIFT

(11) **EP 3 216 853 B1**
(45) Veröffentlichungstag und Bekanntmachung des Hinweises auf die Patenterteilung: **15.05.2019**
(21) Anmeldenummer: 16159673.9
(22) Anmeldetag: 10.03.2016
(51) Int. Cl.: C12M 1/00, C12M 1/34

(54) **TRANSFERVORRICHTUNG**
TRANSFER DEVICE
DISPOSITIF DE TRANSFERT

(43) Veröffentlichungstag der Anmeldung: 13.09.2017
(73) Patentinhaber: Airbus Defence and Space GmbH, 82024 Taufkirchen (DE)
(72) Erfinder: KERN, Dr., Peter, 88682 Salem (DE); HARTSTEIN, Heinz, 88069 Tettnang (DE)
(74) Vertreter: Daub, Thomas

(56) Entgegenhaltungen:
- EP-A1- 0 047 434
- DE-A1- 10 156 349
- US-A- 3 224 143
- US-A- 4 868 123
- US-A- 5 122 255
- US-A1- 2008 220 514

## Beschreibung

### Stand der Technik

Die Erfindung betrifft eine Anlage mit einer Transfervorrichtung.

Es ist bereits eine Anlage mit einer Transfervorrichtung mit zumindest einer Pumpeneinheit, welche zu einem definierten Gastransfer zwischen einem ersten abgeschlossenen Raum einer Anlage und einem von dem ersten Raum getrennten zweiten Raum vorgesehen ist, vorgeschlagen worden. Eine entsprechende Transfervorrichtung wird dabei beispielsweise zu einem Abpumpen von Gas aus dem ersten abgeschlossenen Raum eines Photobioreaktors verwendet, da der kontinuierliche Betrieb des Bioreaktors mit dauernder oder portionsweiser Zugabe von CO₂ zu einem Druckanstieg in dem ersten Raum führt. Dabei wird ein Teil des gesamten Gasgemisches abgepumpt, sodass in der Regel Sauerstoff O₂, Kohlendioxid CO₂ und Stickstoff N₂ aus dem Raum abgepumpt werden.

Die Aufgabe der Erfindung besteht insbesondere darin, eine gattungsgemäße Vorrichtung mit verbesserten Eigenschaften hinsichtlich einer Kontrolle eines, insbesondere selektiven Gastransfers sowie hinsichtlich einer Kontrolle eines Gasverhältnisses bereitzustellen. Die Aufgabe wird erfindungsgemäß durch die Merkmale des Patentanspruchs 1 gelöst, während vorteilhafte Ausgestaltungen und Weiterbildungen der Erfindung den Unteransprüchen entnommen werden können.

### Vorteile der Erfindung

Die Erfindung geht aus von einer Anlage mit einer Transfervorrichtung mit zumindest einer Pumpeneinheit, welche zu einem definierten Gastransfer zwischen einem ersten abgeschlossenen Raum einer Anlage, einer Photobioreaktoreinheit und einem von dem ersten Raum getrennten zweiten Raum vorgesehen ist, sowie einer Steuer- und/oder Regeleinheit gemäß Anspruch 1.

Es wird vorgeschlagen, dass die zumindest eine Pumpeneinheit als eine selektive Sauerstoffpumpe ausgebildet ist. Die Pumpeneinheit ist einer elektrochemischen Sauerstoffpumpe gebildet. Die Transfervorrichtung ist von einer Photobioreaktor-Transfervorrichtung gebildet. Unter einer "Transfervorrichtung" soll in diesem Zusammenhang insbesondere eine Vorrichtung einer Anlage verstanden werden, die zu einer definierten Zufuhr und/oder Abfuhr eines Fluids, insbesondere eines Gases, für eine Anlage vorgesehen ist. Vorzugsweise soll darunter insbesondere eine Vorrichtung verstanden werden, welche zu einem definierten Gastransfer aus zumindest einem abgeschlossenen Raum der Anlage vorgesehen ist. Mittels der Transfervorrichtung kann beispielsweise ein Druckausgleich und/oder ein Übergang, insbesondere ein selektiver Übergang, von Gasen zwischen zumindest zwei voneinander getrennten Räumen erreicht werden. Unter einer "Photobioreaktor-Transfervorrichtung" soll in diesem Zusammenhang insbesondere eine Transfervorrichtung eines Photobioreaktors verstanden werden, die zu einer definierten Zufuhr und/oder Abfuhr eines Fluids, insbesondere eines Gases, für den Photobioreaktor vorgesehen ist. Ferner soll in diesem Zusammenhang unter einer "Pumpeneinheit" insbesondere eine Einheit verstanden werden, die zu einer Förderung zumindest eines Fluids vorgesehen ist. Es soll darunter insbesondere eine Einheit verstanden werden, welche zu einem direkten, definierten Gastransfer zwischen zumindest zwei voneinander getrennten Räumen vorgesehen ist. Ein Gastransfer kann dabei insbesondere unabhängig von einer Druckdifferenz zwischen den zumindest zwei voneinander getrennten Räumen erreicht werden. Des Weiteren soll in diesem Zusammenhang unter einem "abgeschlossenen Raum" insbesondere ein Raum verstanden werden, der in zumindest einem Betriebszustand, vorzugsweise während eines Betriebs der Anlage, zumindest im Wesentlichen gegenüber einer Umgebung hinsichtlich eines freien Luftstroms und der Umgebung abgeschirmt und/oder abgeschlossen ist. Vorzugsweise soll darunter insbesondere ein Raum verstanden werden, bei welchem ungewollte Stoffübergänge aus dem Raum und/oder in den Raum verhindert oder zumindest begrenzt werden. Bevorzugt ist der Raum gegenüber einer Umgebung und/oder zumindest einem angrenzenden Raum hermetisch abgedichtet. Unter einer "Anlage" soll in diesem Zusammenhang insbesondere eine Vorrichtung verstanden werden, welche zumindest einen Reaktor und/oder zumindest einen Prozess umfasst. Bevorzugt soll darunter insbesondere eine Vorrichtung verstanden werden, welche zumindest einen Reaktor für physiochemische, biochemische und/oder biologische Prozesse aufweist. Besonders bevorzugt soll insbesondere eine Vorrichtung verstanden werden, welche zumindest einen Reaktor aufweist, welche bei Prozessen Sauerstoff freisetzen. Unter einer "Photobioreaktoranlage" soll in diesem Zusammenhang insbesondere ein Reaktor oder eine Anlage verstanden werden, welcher oder welche zu einer Produktion und/oder Kultivierung von Mikroorganismen, wie beispielsweise Algen, Cyanobakterien, Moospflanzen, Bakterien, pflanzlichen Zellkulturen und/oder andere Mikroorganismen, insbesondere innerhalb einer künstlichen technischen Umgebung, vorgesehen ist. Vorzugsweise soll darunter insbesondere ein Reaktor oder eine Anlage verstanden werden, welcher oder welche den Prozess der Photosynthese produzierter und/oder kultivierter Mikroorganismen nutzt, um Energie zu gewinnen, insbesondere um mittels Licht Kohlendioxid (CO₂) zu binden und mittels Photosynthese Sauerstoff (O₂) zu erzeugen. Ferner soll in diesem Zusammenhang unter einer "selektiven Sauerstoffpumpe" insbesondere eine bevorzugt für Sauerstoff selektive Pumpeneinheit verstanden werden. Vorzugsweise soll darunter insbesondere eine Pumpeneinheit verstanden werden, mittels welcher selektiv Sauerstoff gepumpt werden kann. Bevorzugt soll darunter insbesondere eine Pumpeneinheit verstanden werden, mittels welcher aus einem Raum unabhängig von einem vorherrschenden Gasgemisch -falls vorhanden- selektiv Sauerstoff abgepumpt und/oder in einen anderen Raum transferiert werden kann. Unter "vorgesehen" soll insbesondere speziell programmiert, ausgelegt und/oder ausgestattet verstanden werden. Darunter, dass ein Objekt zu einer bestimmten Funktion vorgesehen ist, soll insbesondere verstanden werden, dass das Objekt diese bestimmte Funktion in zumindest einem Anwendungs- und/oder Betriebszustand erfüllt und/oder ausführt.

Durch die erfindungsgemäße Ausgestaltung der Transfervorrichtung kann vorteilhaft abhängig von einem Bedarf gezielt und selektiv Sauerstoff transferiert werden. Hierdurch kann insbesondere eine gezielte Kontrolle, Reduktion und/oder Steigerung einer Sauerstoffkonzentration, insbesondere in dem ersten abgeschlossenen Raum der Anlage, insbesondere der Photobioreaktoranlage, erreicht werden. Dadurch kann vorteilhaft eine Kontrolle eines Gastransfers, sowie eine gleichzeitige Kontrolle eines Gasverhältnisses und/oder des Gesamtdrucks erreicht werden. Vorzugsweise kann dadurch eine insbesondere vollkommen elektrisch arbeitende Transfervorrichtung bereitgestellt werden.

Ferner wird vorgeschlagen, dass die zumindest eine Pumpeneinheit zumindest ein Zirkoniumoxidelement aufweist, das zu einem selektiven Transfer von Sauerstoff aus dem ersten Raum in den zweiten Raum vorgesehen ist. Vorzugsweise ist das Zirkoniumoxidelement von einer Zirkoniumoxidkeramik gebildet. Bevorzugt ist das Zirkoniumoxidelement dazu vorgesehen, als lonenleiter und/oder Festkörperelektrolyt zu wirken. Besonders bevorzugt wird ferner eine Pumprate der Pumpeneinheit insbesondere durch eine angelegte Stromstärke und/oder eine Geometrie, insbesondere eine Oberfläche, des Zirkoniumoxidelements beeinflusst. Vorzugsweise kann die Pumpleistung, also ein Volumenstrom pro Zeiteinheit, und die erreichbaren Drücke, insbesondere sowohl auf einer Saug- als auch auf einer Abgabeseite, durch einen Pumpenstrom, eine Betriebstemperatur und die geometrischen Abmessungen der Pumpeneinheit eingestellt und/oder konfiguriert werden. Ferner kann mittels der Transfervorrichtung zudem die Sauerstoffproduktionsrate des Prozesses, insbesondere die Photosyntheserate von Sauerstoff (O₂), oder die Sauerstofftransferrate, bestimmt werden. Bevorzugt ist das Zirkoniumoxidelement zu einem selektiven Transfer von Sauerstoff bei Umgebungsdruck aus dem ersten Raum in den zweiten Raum vorgesehen. Unter einer "Zirkoniumoxidkeramik" soll in diesem Zusammenhang insbesondere eine technische Keramik verstanden werden. Vorzugsweise ist die Zirkoniumoxidkeramik insbesondere von einem nichtmetallischen mineralischen Werkstoff gebildet. Die Zirkoniumoxidkeramik wird insbesondere mit anderen Oxiden, wie beispielsweise Calciumoxid (CaO), Magnesiumoxid (MgO) und/oder Yittriumoxid (Y₂O₃), stabilisiert. Grundsätzlich sind jedoch auch andere, einem Fachmann als sinnvoll erscheinende Keramiken und/oder Elemente denkbar, wie beispielsweise eine Keramik und/oder ein Element welche/welches Titanoxid, Vanadiumoxid, Nioboxid und/oder Perowskit aufweist, wobei die Oxide jeweils wiederum durch andere Oxide stabilisiert sein können. Ein Betriebsdruck des ersten Raums kann zwischen 10⁻³ mbar und 100 bar liegen. Ein Betriebsdruck des zweiten Raums kann ebenfalls zwischen 10⁻³ mbar und 100 bar liegen. Dadurch kann insbesondere eine besonders zuverlässige Pumpeneinheit bereitgestellt werden. Ferner kann dadurch eine vorteilhaft flexible Pumprate der Pumpeneinheit ermöglicht werden. Des Weiteren ist durch einen entsprechenden Aufbau kein periodischer Austausch von Verbrauchsmaterial für den Betrieb notwendig. Zudem wird keine Operatorzeit hierfür benötigt. Ferner kann dadurch ein geringer Ressourcenbedarf erreicht werden. Es kann eine operationell sehr robust, und somit für den Einsatz im Weltraum bevorzugte Transfervorrichtung bereitgestellt werden. Insbesondere kann eine Pumpeneinheit frei von beweglichen Teilen bereitgestellt werden. Vorzugsweise kann eine insbesondere vollkommen elektrisch arbeitende Transfervorrichtung bereitgestellt werden.

Des Weiteren wird vorgeschlagen, dass die zumindest eine Pumpeneinheit zumindest ein Heizelement aufweist, welches dazu vorgesehen ist, das zumindest eine Zirkoniumoxidelement zu erhitzen. Vorzugsweise ist das zumindest eine Heizelement dazu vorgesehen, das Zirkoniumoxidelement auf eine Betriebstemperatur zu erhitzen und insbesondere auf dieser Temperatur zu halten. Bevorzugt ist das zumindest eine Heizelement dazu vorgesehen, das Zirkoniumoxidelement auf eine Betriebstemperatur von zumindest 500 °C, besonders bevorzugt auf eine Betriebstemperatur von 500 °C bis 700 °C, zu erhitzen und insbesondere auf dieser Temperatur zu halten. Unter einem "Heizelement" soll in diesem Zusammenhang insbesondere ein Element verstanden werden, dass zu einer Erzeugung einer Heizleistung vorgesehen ist. Vorzugsweise soll darunter insbesondere ein Element verstanden werden, welches dazu vorgesehen ist, Energie, insbesondere elektrische Energie, in Wärmenergie umzuwandeln. Es sind verschiedene, einem Fachmann als sinnvoll erscheinende Ausgestaltungen eines Heizelements denkbar. Das Heizelement kann beispielsweise als ein Mikrowellen-Heizelement, als ein Widerstandsheizelement und/oder besonders bevorzugt als ein Infrarot-Heizelement ausgebildet sein. Dadurch kann das Zirkoniumoxidelement vorteilhaft auf eine optimale Betriebstemperatur gebracht werden. Ferner kann dadurch ein optimaler Sauerstofftransport ermöglicht werden. Insbesondere kann dadurch eine hohe Effizienz der Pumpeneinheit erreicht werden.

Es wird ferner vorgeschlagen, dass das zumindest eine Zirkoniumoxidelement der zumindest einen Pumpeneinheit von einem dotierten Zirkoniumoxidelement gebildet ist. Vorzugsweise ist das Zirkoniumoxidelement beispielsweise mit Yttriumoxid (Y₂O₃) dotiert. Grundsätzlich wäre jedoch auch denkbar, dass das Zirkoniumoxidelement von einem undotierten Zirkoniumoxidelement und/oder einer Kombination aus dotierten und undotierten Zirkoniumoxidelementen gebildet ist. Dadurch kann eine vorteilhaft effiziente Pumpeneinheit bereitgestellt werden. Ferner kann dadurch insbesondere eine vorteilhaft hohe lonenleitfähigkeit des Zirkoniumoxidelements erreicht werden.

Es wird ferner vorgeschlagen, dass das zumindest eine Zirkoniumoxidelement der zumindest einen Pumpeneinheit, abhängig von einer Polarität eines Pumpenstroms, zu einem bidirektionalen Sauerstoff-Transfer zwischen dem ersten Raum und dem zweiten Raum vorgesehen ist. Vorzugsweise soll darunter insbesondere verstanden werden, dass ein mittels der Pumpeneinheit Sauerstoff sowohl von dem ersten Raum in den zweiten Raum, als auch von dem zweiten Raum in den ersten Raum geführt werden kann. Bevorzugt erfolgt eine Wahl einer Richtung eines Sauerstoffübergangs insbesondere mittels eines Wechsels einer Polarität eines angelegten Potentials. Dadurch kann insbesondere eine vorteilhaft variable Verwendung der Pumpeneinheit erreicht werden. Ferner kann die Pumpeneinheit dadurch vorteilhaft, insbesondere bei gleichem physikalischem Set-up, für einen bidirektionalen Sauerstoff-Transfer zwischen dem ersten Raum und dem zweiten Raum verwendet werden.

Ferner ist die zumindest eine Pumpeneinheit zu dem gezielten, selektiven Abtrennen von Sauerstoff vorgesehen. Dadurch kann insbesondere ein gezieltes und selektives Transferieren von Sauerstoff ermöglicht werden. Es kann insbesondere vorteilhaft eine Kontrolle eines Gastransfers sowie eine gleichzeitige Kontrolle eines Partialdrucks und/oder des Gesamtdrucks erreicht werden.

Es wird weiter vorgeschlagen, dass die zumindest eine Pumpeneinheit in zumindest einem Betriebszustand zu einer elektrischen Reduktion zumindest eines Gases vorgesehen ist. Vorzugsweise ist die zumindest eine Pumpeneinheit in zumindest einem Betriebszustand zu einer elektrischen Reduktion zumindest eines Gases in dem ersten Raum vorgesehen. Dadurch kann eine vorteilhaft effiziente Pumpeneinheit bereitgestellt werden.

Des Weiteren wird vorgeschlagen, dass die zumindest eine Pumpeneinheit zu einer Anreicherung eines reduzierten Gases in dem ersten Raum und/oder dem zweiten Raum vorgesehen ist. Vorzugsweise ist die zumindest eine Pumpeneinheit zu einer Anreicherung eines reduzierten Gases in dem ersten Raum vorgesehen. Hierdurch kann insbesondere eine gezielte und selektive Gasanreicherung ermöglicht werden.

Es wird weiter vorgeschlagen, dass die Transfervorrichtung zumindest eine Messeinheit aufweist, welche dazu vorgesehen ist, eine Spannungsdifferenz zwischen zumindest zwei Seiten der Pumpeneinheit zu erfassen. Vorzugsweise ist die Messeinheit dazu vorgesehen, eine Spannungsdifferenz zwischen einer ersten Seite der Pumpeneinheit, welche in dem ersten Raum angeordnet ist, und einer zweiten Seite der Pumpeneinheit, welche in dem zweiten Raum angeordnet ist, zu erfassen. Bevorzugt weist die Messeinheit zumindest einen Spannungsmesser auf, welcher zu einer Erfassung einer Spannungsdifferenz zwischen zumindest zwei Kontaktpunkten, insbesondere zwischen zumindest zwei Elektroden, vorgesehen ist. Besonders bevorzugt umfasst die Messeinheit zudem eine Recheneinheit, welche zu einer Auswertung von Messsignalen des Spannungsmessers vorgesehen ist. Unter einer "Recheneinheit" soll insbesondere eine Einheit mit einem Informationseingang, einer Informationsverarbeitung und einer Informationsausgabe verstanden werden. Vorteilhaft weist die Recheneinheit zumindest einen Prozessor, einen Speicher, Ein- und Ausgabemittel, weitere elektrische Bauteile, ein Betriebsprogramm, Regelroutinen, Steuerroutinen und/oder Berechnungsroutinen auf. Grundsätzlich kann die Recheneinheit jedoch auch als eine Logikschaltung, wie beispielsweise als ein FPGA (Field Programmable Gate Array), und/oder eine Analogschaltung ausgebildet sein. Dadurch kann die Pumpeneinheit insbesondere zusätzlich zu einer Erfassung von Daten verwendet werden. Vorzugsweise kann die Pumpeneinheit dadurch eine zusätzliche Überwachungsfunktion übernehmen.

Ferner wird vorgeschlagen, dass die zumindest eine Messeinheit dazu vorgesehen ist, abhängig von der Spannungsdifferenz auf eine Druckdifferenz zwischen dem ersten Raum und dem zweiten Raum zu schließen. Die zumindest eine Messeinheit ist dazu vorgesehen, abhängig von der Spannungsdifferenz auf eine Partialdruckdifferenz zwischen dem ersten Raum und dem zweiten Raum zu schließen. Besonders bevorzugt kann eine vorliegende Sauerstoffkonzentration und/oder Unterschiede der Sauerstoffkonzentrationen bestimmt werden, insbesondere wenn ein Sauerstoffpartialdruck in dem ersten Raum und/oder dem zweiten Raum bekannt sind. Dadurch kann die Transfervorrichtung vorteilhaft zu einer Drucküberwachung verwendet werden.

Die Erfindung geht aus von einer Photobioreaktoranlage, mit einer Transfervorrichtung. Die Anlage weist eine Steuer- und/oder Regeleinheit auf, welche einen absoluten Sauerstoffpartialanteil in dem ersten Raum außerhalb eines Reaktionsbereichs mittels der Transfervorrichtung steuert und/oder regelt. Vorzugsweise ist die Steuer- und/oder Regeleinheit dazu vorgesehen, einen absoluten Sauerstoffpartialanteil in dem ersten Raum außerhalb eines Reaktionsbereichs auf einen Wert von maximal 25%, vorzugsweise von maximal 20% und besonders bevorzugt von maximal 15% zu steuern und/oder zu regeln. Grundsätzlich sind jedoch, insbesondere je nach Anwendung, auch andere, einem Fachmann als sinnvoll erscheinende Werte des Sauerstoffpartialanteils denkbar. Bevorzugt ist die Steuer- und/oder Regeleinheit dazu vorgesehen, einen absoluten Sauerstoffpartialanteil in dem ersten Raum außerhalb eines Reaktionsbereichs auf einen Wert von annähernd 15% zu steuern und/oder zu regeln. Besonders bevorzugt ist die Steuer- und/oder Regeleinheit dazu vorgesehen, einen absoluten Sauerstoffpartialanteil in dem ersten Raum außerhalb eines Reaktionsbereichs auf einen Wert zu steuern und/oder zu regeln, sodass ein betragsmäßiges equimolares Verhältnis von dCO₂/dt = - dO₂/dt erreicht wird. Die Steuer- und/oder Regeleinheit steuert und/oder regelt einen absoluten Sauerstoffpartialanteil in dem ersten Raum außerhalb eines Reaktionsbereichs durch Abpumpen von Sauerstoff mittels der Transfervorrichtung. Unter einer "Steuer- und/oder Regeleinheit" soll insbesondere eine Einheit mit zumindest einer Steuerelektronik verstanden werden. Unter einer "Steuerelektronik" soll insbesondere eine Einheit mit einer Prozessoreinheit und mit einer Speichereinheit sowie mit einem in der Speichereinheit gespeicherten Betriebsprogramm verstanden werden. Grundsätzlich kann die Steuerelektronik jedoch auch als eine Logikschaltung, wie beispielsweise als ein FPGA (Field Programmable Gate Array), und/oder eine Analogschaltung ausgebildet sein. Dadurch kann ein vorteilhaftes Gasgemisch in dem ersten Raum der Anlage, insbesondere der Photobioreaktoranlage, bereitgestellt werden. Vorzugsweise kann dadurch ein absoluter Sauerstoffpartialanteil in dem ersten Raum vorteilhaft auf einen Wert gesteuert und/oder geregelt werden, bei welchem eine vorteilhafte, insbesondere optimale O₂-Produktionsrate und/oder O₂-Abgaberate der Anlage, insbesondere der Photobioreaktoranlage, vorliegt. Dadurch kann insbesondere eine vollständige Integration der Transfervorrichtung in ein automatisches, insbesondere rechnerbasiertes Steuer- und/oder Kontrollkonzept ermöglicht werden.

Die Photobioreaktoranlage weist zumindest eine Photobioreaktoreinheit auf, welche zugeführtes Kohlendioxid aufnimmt und mittels Photosynthese Sauerstoff erzeugt und abgibt. einer "Photobioreaktoreinheit" soll in diesem Zusammenhang insbesondere eine Einheit verstanden werden, welche zu einer direkten Produktion und/oder Kultivierung von Mikroorganismen wie beispielsweise Algen, Cyanobakterien, Moospflanzen und/oder pflanzlichen Zellkulturen, insbesondere innerhalb einer künstlichen technischen Umgebung, vorgesehen ist. Vorzugsweise soll darunter insbesondere eine Einheit verstanden werden, welche den Prozess der Photosynthese produzierter und/oder kultivierter Mikroorganismen direkt nutzt, um mittels Photosynthese mit Licht, Kohlendioxid (CO₂) und Wasser den Sauerstoff (O₂) zu erzeugen. Bevorzugt weist die Einheit dazu eine Lichtquelle zu einer Bestrahlung der Mikroorganismen auf. Dadurch kann eine vorteilhafte Anlage bereitgestellt werden.

Ferner geht die Erfindung aus von einem Verfahren zum Betrieb der Anlage, insbesondere der Photobioreaktoranlage. Es wird vorgeschlagen, dass mittels der Transfervorrichtung ein absoluter Sauerstoffpartialanteil in dem ersten Raum außerhalb eines Reaktionsbereichs gesteuert und/oder geregelt wird. Vorzugsweise wird der absolute Sauerstoffpartialanteil in dem ersten Raum außerhalb eines Reaktionsbereichs mittels der Transfervorrichtung auf einen Wert von maximal 25%, vorzugsweise von maximal 20% und besonders bevorzugt von maximal 15% gesteuert und/oder geregelt. Besonders bevorzugt wird der absolute Sauerstoffpartialanteil in dem ersten Raum außerhalb eines Reaktionsbereichs mittels der Transfervorrichtung auf einen Wert von annähernd 15% gesteuert und/oder geregelt. Besonders bevorzugt wird der absolute Sauerstoffpartialanteil in dem ersten Raum außerhalb eines Reaktionsbereichs mittels der Transfervorrichtung auf einen Wert gesteuert und/oder geregelt, sodass ein betragsmäßiges equimolares Verhältnis von dCO₂/dt = - dO₂/dt erreicht wird. Dadurch kann ein vorteilhaftes Gasgemisch in dem ersten Raum der Anlage, insbesondere der Photobioreaktoranlage, bereitgestellt werden. Vorzugsweise kann dadurch ein absoluter Sauerstoffpartialanteil in dem ersten Raum vorteilhaft auf einen Wert gesteuert und/oder geregelt werden, bei welchem eine vorteilhafte, insbesondere optimale O₂-Produktionsrate der Anlage vorliegt.

Des Weiteren wird vorgeschlagen, dass die Transfervorrichtung zu einer Überwachung und/oder zu einer Einstellung zumindest eines sicherheitsrelevanten Betriebszustand verwendet wird. Vorzugsweise kann die Transfervorrichtung beispielsweise zu einer Überwachung und/oder zu einer Einstellung eines Drucks und/oder einer Sauerstoffkonzentration in dem zweiten Raum und/oder besonders bevorzugt in dem ersten Raum verwendet werden. Dadurch kann insbesondere eine vorteilhaft vielfältige Nutzung der Transfervorrichtung erreicht werden. Vorzugsweise kann die Transfervorrichtung dadurch vorteilhaft eine über einen Transfer hinausgehende Funktion erfüllen.

Es wird ferner vorgeschlagen, dass die Durchführung unter Bedingungen reduzierter oder erhöhter Schwerkraft erfolgt. Vorzugsweise soll dieses Verfahren im Weltraum angewandt werden, wie beispielsweise bei µg in einem Raumschiff, einem Prozess in einem Raumschiff bei Beschleunigungen von 10⁻⁶ xg bis 10 xg, auf einem Planeten, wie dem Mars, und/oder auf einem Trabanten, wie dem Mond. Die g-Werte sind dabei insbesondere auf einem Planeten und/oder einem Asteroiden oder in einem fliegenden Raumschiff zu verstehen. Grundsätzlich kann jedoch ein g-Wert verfahrenstechnisch drastisch erhöht werden, wie beispielsweise auf 100 xg. Beispielsweise können eine Anlage und/oder ein Reaktor einer künstlichen Prozessbeschleunigung ausgesetzt sein, welche von den angegebenen g-Werten abweicht. Unter "Bedingungen reduzierter Schwerkraft" sollen insbesondere Bedingungen verstanden werden, bei denen eine Schwerewirkung von maximal 0,9 xg, vorteilhaft von minimal bis 1*10⁻³ xg, vorzugsweise von minimal bis 1*10⁻⁶ xg und besonders bevorzugt von minimal bis 1*10⁻⁸ xg, wirksam ist. Ferner sollen unter "Bedingungen erhöhter Schwerkraft" insbesondere Bedingungen verstanden werden, bei denen eine Schwerewirkung von zumindest 1,1 xg, vorzugsweise bis maximal 10 xg, wirksam ist. Die Schwerewirkung kann durch Gravitation und/oder künstlich durch eine Beschleunigung erzeugt sein. Die g-Werte können grundsätzlich verfahrenstechnisch drastisch erhöht werden. Mit "g" ist der Wert der Fallbeschleunigung auf der Erde von 9,81 m/s² bezeichnet. Das Verfahren kann vorteilhaft in Lebenserhaltungssystemen und/oder in Klimaanlagen im Weltraum angewendet werden, wie beispielsweise bei Raumfahrzeugen, Habitaten im Weltraum, Gewächshäusern, bemannten System, wie dem Rover, und/oder bei Raumanzügen, insbesondere bei EVA-Anzügen (Extra Vehicular Activity-Anzügen).

Es wird weiter vorgeschlagen, dass das Verfahren bei einer ISRU-Anwendung durchgeführt wird. Das Verfahren kann dabei zu der Abtrennung und/oder Anreicherung von Sauerstoff aus einem Gasgemisch und/oder aus lokal vorliegendem Material, wie beispielsweise einem lokal vorliegenden Festkörper, im Rahmen von In-Situ Ressource Utilisation (ISRU) Anwendungen auf Planeten angewendet werden. Ferner kann das Verfahren alternativ auch in geschlossenen Kompartments in terrestrischen Anwendungen angewendet werden, wie beispielsweise bei Flugzeugen, U-Booten, Bunker, hermetisch abgeschlossenen Räumen, Gewächshäusern und/oder Fahrzeugen, wie Autos, Züge, etc. Dadurch kann eine vorteilhafte Anwendung des Verfahrens erreicht werden.

Ferner wird vorgeschlagen, dass das Verfahren als Teil eines Environmental Control Systems eingesetzt wird. Das Verfahren wird dabei insbesondere zu einer Druck- und/oder Temperaturregelung und/oder zu einer Spaltung von Gasen, z.B. Kohlendioxid (CO₂) zur Gewinnung von Sauerstoff (O₂) und/oder zu einer kontrollierten Sauerstoffversorgung und/oder -regelung verwendet. Vorzugsweise wird das Verfahren zu einer Druck- und Temperaturregelung sowie zu einer Sauerstoffversorgung in zumindest im Wesentlichen geschlossenen Systemen, wie insbesondere in Weltraum-, Luft-, Land- oder Wasserfahrzeugen, verwendet. Das Verfahren kann dabei insbesondere sowohl in einem Environmental Control System auf der Erde als auch im Weltraum, in Raumfahrzeugen und/oder auf Planeten und/oder Asteroiden, durchgeführt werden. Unter einem "Environmental Control System" soll in diesem Zusammenhang insbesondere ein Umgebungskontrollsystem verstanden werden. Vorzugsweise soll darunter insbesondere ein Klimasystem verstanden werden, welches zu einem Luftaustausch, einer Druckregelung, einer Temperaturregelung, einer Druckversorgung und/oder einer Sauerstoffversorgung, insbesondere in einer zumindest im Wesentlichen geschlossenen Kabine, vorgesehen ist. Bevorzugt soll darunter insbesondere ein Klimasystem eines Raumfahrzeugs und/oder eines Flugzeugs, insbesondere eines Verkehrsflugzeugs, verstanden werden. Dadurch kann eine vorteilhafte Anwendung des Verfahrens erreicht werden.

Die erfindungsgemäße Transfervorrichtung, die Anlage sowie das Verfahren sollen hierbei nicht auf die oben beschriebene Anwendung und Ausführungsform beschränkt sein. Insbesondere können die erfindungsgemäße Transfervorrichtung, die Anlage sowie das Verfahren zu einer Erfüllung einer hierin beschriebenen Funktionsweise eine von einer hierin genannten Anzahl von einzelnen Elementen, Bauteilen und Einheiten abweichende Anzahl aufweisen.

### Zeichnungen

Weitere Vorteile ergeben sich aus der folgenden Zeichnungsbeschreibung. In den Zeichnungen ist ein Ausführungsbeispiel der Erfindung dargestellt. Die Zeichnungen, die Beschreibung und die Ansprüche enthalten zahlreiche Merkmale in Kombination. Der Fachmann wird die Merkmale zweckmäßigerweise auch einzeln betrachten und zu sinnvollen weiteren Kombinationen zusammenfassen.

Es zeigen:
- Fig. 1: eine Anlage mit einer Photobioreaktoreinheit, einer Steuer- und Regeleinheit und einer Transfervorrichtung in einer schematischen Darstellung,
- Fig. 2: die Transfervorrichtung mit einer Pumpeneinheit, welche ein Zirkoniumoxidelement aufweist, in einer schematischen Darstellung,
- Fig. 3: einen Teilausschnitt III-III aus Fig. 2 der Transfervorrichtung in einer schematischen Darstellung und
- Fig. 4: ein Diagramm von Gaskonzentrationen über die Zeit während eines beispielhaften Betriebs der Anlage mit einem Algenreaktor.

### Beschreibung des Ausführungsbeispiels

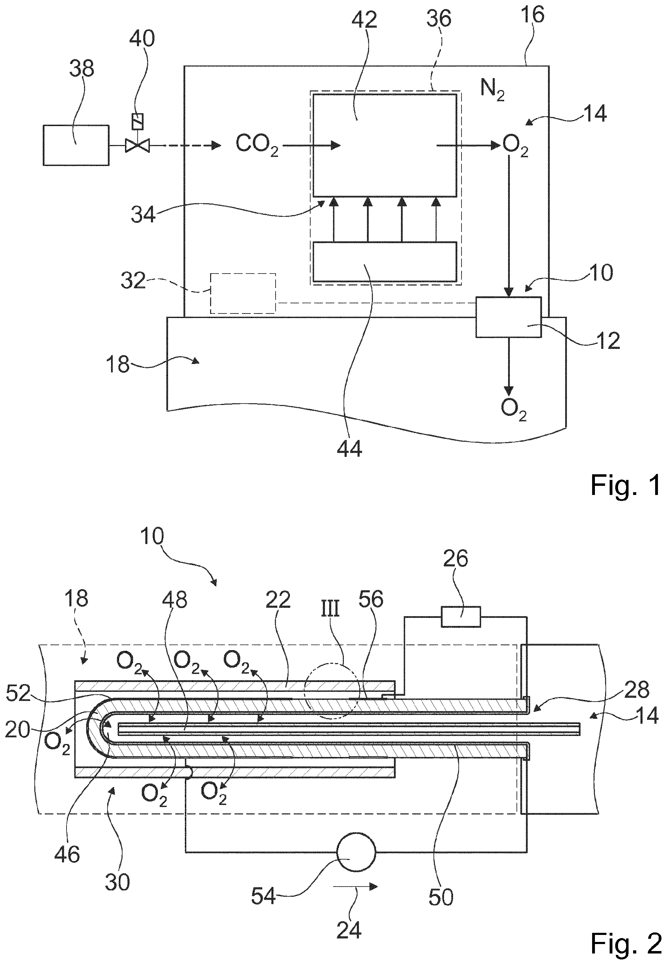

Figur 1 zeigt eine Anlage 16. Die Anlage 16 ist von einer Photobioreaktoranlage gebildet. Die Anlage 16 ist für eine Raumfahranwendung vorgesehen. Die Anlage 16 weist einen Raum 14 auf. Der Raum 14 ist von einem abgeschlossenen Raum gebildet. Ferner weist die Anlage 16 eine Photobioreaktoreinheit 36 auf. Grundsätzlich kann die Anlage 16 auch mehrere Photobioreaktoreinheiten 36 aufweisen. Ferner kann die Anlage 16 grundsätzlich auch andere, einem Fachmann als sinnvoll erscheinende Reaktoren aufweisen. Die Photobioreaktoreinheit 36 ist in dem Raum 14 angeordnet. Die Photobioreaktoreinheit 36 ist dazu vorgesehen, zugeführtes Kohlendioxid CO₂ aufzunehmen und mittels Photosynthese, unter Nutzung einer Lichtquelle 44, Sauerstoff O₂ zu erzeugen und abzugeben. Die Anlage 16 weist einen Gasspeicher 38 auf. Der Gasspeicher 38 ist von einem Kohlendioxid-Speicher gebildet. Der Gasspeicher 38 ist über eine Leitung mit einem Ventil 40 mit dem Raum 14 verbunden. Das Ventil 40 ist beispielhaft von einem Magnetventil gebildet. Aus dem Gasspeicher 38 wird für eine Reaktion der Photobioreaktoreinheit 36 Kohlendioxid CO₂ in den Raum 14 geleitet. Eine Menge des Kohlendioxids CO₂ wird dabei über das Ventil 40 gesteuert.

Die Photobioreaktoreinheit 36 ist zu einer Produktion und/oder Kultivierung von Mikroorganismen wie beispielsweise Algen, Cyanobakterien, Moospflanzen und/oder pflanzlichen Zellkulturen innerhalb einer künstlichen technischen Umgebung vorgesehen. Die Photobioreaktoreinheit 36 weist als photosynthetisches System beispielhaft eine Algenkultur auf. Die Photobioreaktoreinheit 36 ist dazu vorgesehen, den Prozess der Photosynthese produzierter und/oder kultivierter Mikroorganismen zu nutzen, um aus Licht, Kohlendioxid CO₂ und Wasser mittels Photosynthese Sauerstoff O₂ zu erzeugen. Die Photobioreaktoreinheit 36 weist ein Aufnahmeelement 42 zur Aufnahme der Mikroorganismen auf. Das Aufnahmeelement 42 kann beispielsweise von einer Rohrleitung, einer Platte und/oder einem anderen, einem Fachmann als sinnvoll erscheinenden Aufnahmeelement 42 gebildet sein. Ferner weist die Photobioreaktoreinheit 36 eine Lichtquelle 44 auf. Die Lichtquelle 44 ist von einer Kunstlichtquelle gebildet. Grundsätzlich wäre jedoch auch denkbar, dass Sonnenlicht als Lichtquelle verwendet wird. Die Lichtquelle 44 ist während eines Betriebs zu einer Bestrahlung der Mikroorganismen vorgesehen. Die Photobioreaktoreinheit 36 bildet einen einen Reaktionsbereich 34 der Anlage 16.

Des Weiteren weist die Anlage 16 eine Transfervorrichtung 10 auf. Die Transfervorrichtung 10 ist von einer Photobioreaktor-Transfervorrichtung gebildet. Die Transfervorrichtung 10 ist zwischen dem ersten Raum 14 der Anlage 16 und einem zweiten Raum 18 angeordnet. Der zweite Raum 18 bildet eine Umgebung der Anlage 16. Der zweite Raum 18 ist von dem ersten Raum 14 der Anlage 16 getrennt ausgebildet. Der zweite Raum 18 ist in diesem Ausführungsbeispiel von der Kabine einer Raumstation gebildet. Die Transfervorrichtung 10 grenzt sowohl an den ersten Raum 14 als auch an den zweiten Raum 18 an. Ein Betriebsdruck des ersten Raums 14 kann zwischen 10⁻³ mbar und 100 bar liegen. Ein Betriebsdruck des zweiten Raums 18 kann ebenfalls zwischen dem Druck des ersten Raums 14 und 100 bar liegen. Vorzugsweise erfolgt ein Betrieb der Transfervorrichtung 10 bei Umgebungsdruck, bevorzugt im Bereich von 750 mbar bis 1250 mbar in dem ersten Raum 14 und im Bereich von 800 mbar bis 1500 mbar in dem zweiten Raum 18.

Ferner weist die Transfervorrichtung 10 eine Pumpeneinheit 12 auf. Die Pumpeneinheit 12 ist zu einem definierten Gastransfer zwischen dem ersten abgeschlossenen Raum 14 der Anlage 16 und dem von dem ersten Raum 14 getrennten zweiten Raum 18 vorgesehen. Die Pumpeneinheit 12 ist als eine selektive Sauerstoffpumpe ausgebildet. Die Pumpeneinheit 12 ist von einer elektrochemischen Sauerstoffpumpe gebildet. Des Weiteren weist die Pumpeneinheit 12 ein Zirkoniumoxidelement 20 auf. Das Zirkoniumoxidelement 20 ist zu einem selektiven Transfer von Sauerstoff O₂ aus dem ersten Raum 14 in den zweiten Raum 18 vorgesehen. Das Zirkoniumoxidelement 20 ist von einer Zirkoniumoxidkeramik gebildet. Das Zirkoniumoxidelement 20 der Pumpeneinheit 12 ist von einem dotierten Zirkoniumoxidelement gebildet. Das Zirkoniumoxidelement 20 ist mit Yttriumoxid (Y₂O₃) dotiert. Grundsätzlich wäre jedoch auch denkbar, dass das Zirkoniumoxidelement 20 von einem undotierten Zirkoniumoxidelement gebildet ist. Das Zirkoniumoxidelement 20 ist dazu von einem Zirkoniumoxidröhrchen gebildet, welches an einer Seite geschlossen ist. Das Zirkoniumoxidelement 20 ist "reagenzglasförmig" ausgebildet. Grundsätzlich ist jedoch auch eine andere, einem Fachmann als sinnvoll erscheinende Formgebung denkbar. Beispielsweise ist auch eine flächige Ausgestaltung des Zirkoniumoxidelements 20 denkbar, wobei die Fläche hierbei eine Trennebene zwischen den beiden Räumen 14, 18 ausbildet. Das Zirkoniumoxidelement 20 bildet daher einen Hohlraum 46 aus. Das Zirkoniumoxidelement 20 ragt mit einer geschlossenen Seite in den zweiten Raum 18. Eine geöffnete Seite des Zirkoniumoxidelements 20 ragt in den ersten Raum 14. Grundsätzlich wäre jedoch auch denkbar, dass das Zirkoniumoxidelement 20 mit einer geschlossenen Seite in den ersten Raum 14 ragt und eine geöffnete Seite des Zirkoniumoxidelements 20 in den zweiten Raum 18 ragt. Der Hohlraum 46 des Zirkoniumoxidelements 20 ist mit dem ersten Raum 14 verbunden. Ferner weist die Pumpeneinheit 12 eine Führungsrohr 48 auf, welches aus dem ersten Raum 14 in den Hohlraum 46 ragt. Das Führungsrohr 48 dient zu einer Führung von in den Hohlraum 46 strömendem Gas. Das Führungsrohr 48 erstreckt sich parallel zu dem Zirkoniumoxidelement 20. Das Führungsrohr 48 ist zu dem Zirkoniumoxidelement 20 beabstandet. Das Führungsrohr 48 erstreckt sich bis kurz vor eine geschlossene Seite des Zirkoniumoxidelements 20. Über das Führungsrohr 48 kann Gas in den Hohlraum 46 einströmen. In dem Hohlraum 46 befindliches Gas ist dazu vorgesehen, in einer Lücke des Hohlraums 46, zwischen dem Führungsrohr 48 und dem Zirkoniumoxidelement 20 vorbei aus dem Hohlraum 46 zu strömen. Hierdurch kann eine vorteilhafte Zirkulation erreicht werden. Ferner kann eine vorteilhaft gleichmäßige Durchströmung des Hohlraums 46 erreicht werden (Fig. 2).

Des Weiteren weist die Pumpeneinheit 12 zwei Elektroden 50, 52 auf. Die Elektroden 50, 52 bestehen zumindest teilweise aus Platin. Die Elektroden 50, 52 sind jeweils von einer strukturierten Elektrode mit Freistellen zwischen dem Elektrodenmaterial gebildet. Die Elektroden 50, 52 dienen zusätzlich als Katalysator zur Dissoziation von sauerstoffhaltigen Gasen, wie beispielsweise SO₂, CO₂, NO, NO₂ oder O₃, und/oder zur selektiven Abtrennung von Sauerstoff O₂. Grundsätzlich wäre jedoch auch eine andere, einem Fachmann als sinnvoll erscheinende Ausgestaltung der Elektroden denkbar. Insbesondere sind andere Elektrodenmaterialien denkbar, wie beispielsweise Palladium, Rhodium, Cobalt, Iridium und/oder Nickel. Grundsätzlich wäre auch die Verwendung eines separaten, von den Elektroden 50, 52 getrennten Katalysators denkbar. Eine erste Elektrode 50 der Pumpeneinheit 12 ist auf einer dem Hohlraum 46 zugewandten Innenseite des Zirkoniumoxidelements 20 angeordnet. Die erste Elektrode 50 erstreckt sich über einen Großteil einer Innenseite des Zirkoniumoxidelements 20. Die erste Elektrode 50 ist zwischen dem Zirkoniumoxidelement 20 und dem ersten Raum 14 angeordnet. Eine zweite Elektrode 52 der Pumpeneinheit 12 ist auf einer dem Hohlraum 46 abgewandten Außenseite des Zirkoniumoxidelements 20 angeordnet. Die zweite Elektrode 52 der Pumpeneinheit 12 ist auf einer, dem geöffneten Ende des Zirkoniumoxidelements 20 abgewandten Ende des Zirkoniumoxidelements 20, auf einer Außenseite des Zirkoniumoxidelements 20 angeordnet. Auf einer dem geöffneten Ende des Zirkoniumoxidelements 20 abgewandten Seite des Zirkoniumoxidelements 20 ist das Zirkoniumoxidelement 20 zu einem großen Teil von der zweiten Elektrode 52 umgeben. Die zweite Elektrode 52 ist zwischen dem Zirkoniumoxidelement 20 und dem zweiten Raum 18 angeordnet. Die Elektroden 50, 52 sind über Leitungen mit einer Energiequelle 54 verbunden. Die Energiequelle 54 bildet eine gemeinsame Spannungsquelle der Elektroden 50, 52. Die Energiequelle 54 stellt einen Pumpenstrom bereit. Das Zirkoniumoxidelement 20 der Pumpeneinheit 12 ist abhängig von einer Polarität 24 des Pumpenstroms zu einem bidirektionalen Sauerstoff-Transfer zwischen dem ersten Raum 14 und dem zweiten Raum 18 vorgesehen (Fig. 2).

Ferner weist die Pumpeneinheit 12 ein Heizelement 22 auf. Das Heizelement 22 ist dazu vorgesehen, das Zirkoniumoxidelement 20 zu erhitzen. Das Heizelement 22 ist dazu vorgesehen, das Zirkoniumoxidelement 20 auf eine Betriebstemperatur zu erhitzen und auf dieser zu halten. Das Heizelement 22 ist dazu vorgesehen, das Zirkoniumoxidelement 20 auf eine Betriebstemperatur zwischen 500 °C und 700 °C zu erhitzen und auf dieser zu halten. Das Heizelement 22 ist von einem Infrarotheizstrahler gebildet. Grundsätzlich wäre jedoch auch eine andere, einem Fachmann als sinnvoll erscheinende Ausbildung des Heizelements 22 denkbar, wie insbesondere als Widerstandsheizer. Das Heizelement 22 ist rohrförmig ausgebildet. Grundsätzlich wäre jedoch auch eine andere, einem Fachmann als sinnvoll erscheinende Ausbildung denkbar. Beispielsweise könnte das Heizelement 22 bei einer flachen, insbesondere plattenförmigen Ausgestaltung des Zirkoniumoxidelements 20 grundsätzlich auch aus zwei Teilheizelementen bestehen, welche jeweils auf gegenüberliegenden Seiten des Zirkoniumoxidelements 20 angeordnet sind. Das Heizelement 22 umgreift das Zirkoniumoxidelement 20 an seiner Mantelfläche im Bereich der zweiten Elektrode 52. Das Heizelement 22 erstreckt sich parallel zu einer Haupterstreckungsrichtung des Zirkoniumoxidelements 20. Das Heizelement 22 ist zu dem Zirkoniumoxidelement 20 sowie zu der zweiten Elektrode 52 beabstandet (Fig. 2).

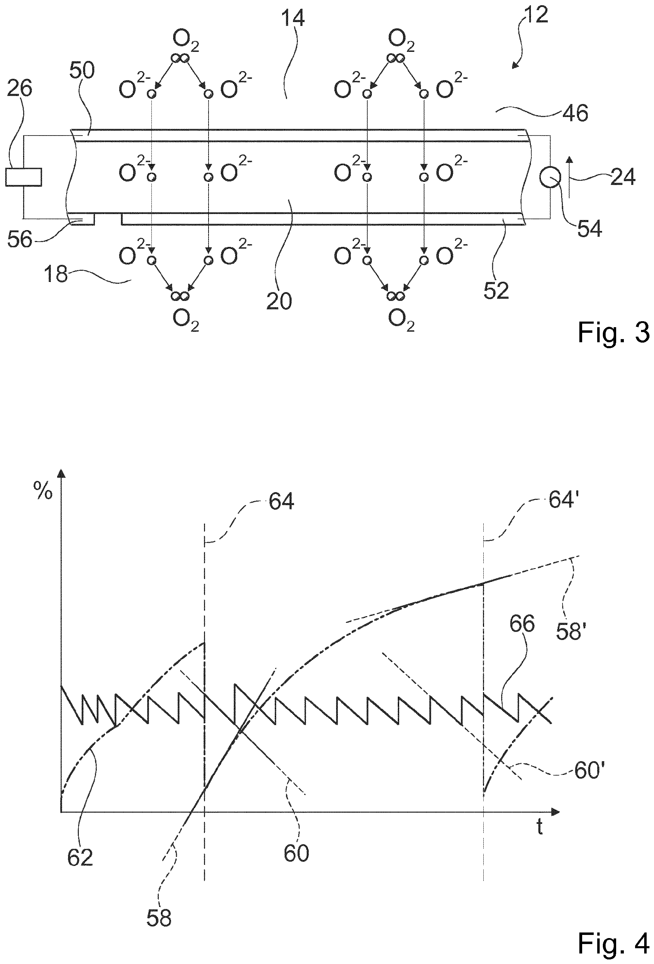

Das Zirkoniumoxidelement 20 wirkt als selektive Sauerstoffpumpe, welche nur Sauerstoff O₂ transportiert, wenn das Zirkoniumoxidelement 20 auf Betriebstemperatur ist, und ein Pumpstrom zwischen einer Innenseite und einer Außenseite des Zirkoniumoxidelements 20 fließt. Die Pumpeneinheit 12 ist dabei zu einer elektrischen Reduktion zumindest eines Gases vorgesehen. Bei diesen Betriebsbedingungen wirkt das Zirkoniumoxidelement 20 als lonenleiter und/oder Festkörperelektrolyt. Die Wirkungsweise der Pumpeneinheit 12 ist, dass Sauerstoff O₂ selektiv aus einem Gasgemisch in dem ersten Raum 14 in den zweiten Raum 18 gepumpt wird. Die Pumpeneinheit 12 ist dabei zu dem gezielten, selektiven Abtrennen von Sauerstoff O₂ vorgesehen. Grundsätzlich kann die Pumpeneinheit 12 damit jedoch auch zu einer gezielten Anreicherung eines reduzierten Gases in dem ersten Raum 14 oder dem zweiten Raum 18 vorgesehen genutzt werden (Fig. 2, 3).

Die Transfervorrichtung 10 weist ferner eine Messeinheit 26 auf. Die Messeinheit 26 ist dazu vorgesehen, eine Spannungsdifferenz zwischen zwei Seiten 28, 30 der Pumpeneinheit 12 zu erfassen. Die Messeinheit 26 ist dazu vorgesehen, eine Spannungsdifferenz zwischen einer ersten, dem ersten Raum 14 zugewandten Seite 28 und einer zweiten, dem zweiten Raum 18 zugewandten Seite 30 der Pumpeneinheit 12 zu erfassen. Die Messeinheit 26 weist dazu einen Spannungsmesser auf. Die Messeinheit 26 ist über eine Leitung mit der ersten Elektrode 50 der Pumpeneinheit 12 verbunden. Ferner ist die Messeinheit 26 über eine Leitung mit einer dritten Elektrode 56 verbunden. Die dritte Elektrode 56 ist auf einer dem Hohlraum 46 abgewandten Außenseite des Zirkoniumoxidelements 20 angeordnet. Grundsätzlich wäre jedoch auch eine andere, einem Fachmann als sinnvoll erscheinende Anordnung der dritten Elektrode 56 denkbar. Die dritte Elektrode 56 ist zwischen dem geöffneten Ende des Zirkoniumoxidelements 20 und der zweiten Elektrode 52, auf einer Außenseite des Zirkoniumoxidelements 20 angeordnet. Die dritte Elektrode 56 ist gegenüber der zweiten Elektrode 52 separiert und weist keine elektrische Verbindung zu der zweiten Elektrode 52 auf. Die dritte Elektrode 56 ist ringförmig ausgebildet und erstreckt sich um das Zirkoniumoxidelement 20. Die dritte Elektrode 56 ist zwischen dem Zirkoniumoxidelement 20 und dem zweiten Raum 18 angeordnet. Die Messeinheit 26 ist dazu vorgesehen, eine Spannungsdifferenz zwischen der ersten Elektrode 50 und der dritten Elektrode 56 zu erfassen. Die Messeinheit 26 ist dazu vorgesehen, abhängig von der Spannungsdifferenz auf eine Druckdifferenz zwischen dem ersten Raum 14 und dem zweiten Raum 18 zu schließen. Die Messeinheit 26 ist dazu vorgesehen, abhängig von der Spannungsdifferenz auf eine Partialdruckdifferenz zwischen dem ersten Raum 14 und dem zweiten Raum 18 zu schließen. Ferner kann eine vorliegende Sauerstoffkonzentration und/oder Unterschiede der Sauerstoffkonzentrationen der Räume 14, 18 bestimmt werden, insbesondere wenn ein Sauerstoffpartialdruck in dem ersten Raum 14 und/oder dem zweiten Raum 18 bekannt sind (Fig. 2).

Ferner weist die Anlage 16 eine Steuer- und Regeleinheit 32 auf. Die Steuer- und Regeleinheit 32 weist eine Steuerelektronik auf. Die Steuer- und Regeleinheit 32 ist dazu vorgesehen, einen absoluten Sauerstoffpartialanteil in dem ersten Raum 14 außerhalb des Reaktionsbereichs 34 mittels der Transfervorrichtung 10 zu regeln. Die Steuer- und Regeleinheit 32 ist dazu vorgesehen, einen absoluten Sauerstoffpartialanteil in dem ersten Raum 14 außerhalb eines Reaktionsbereichs 34 auf einen Wert von annähernd 15% zu regeln. Die Steuer- und Regeleinheit 32 ist zu einer Überwachung des absoluten Sauerstoffpartialanteils vorgesehen. Die Steuer- und Regeleinheit 32 nutzt zu einer Überwachung des absoluten Sauerstoffpartialanteils die Messeinheit 26 der Transfervorrichtung 10. Grundsätzlich wäre jedoch auch denkbar, dass die Steuer- und Regeleinheit 32 einen separaten Sensor zu einer Überwachung des absoluten Sauerstoffpartialanteils aufweist. Die Steuer- und Regeleinheit 32 ist dazu vorgesehen, einen absoluten Sauerstoffpartialanteil in dem ersten Raum 14 außerhalb eines Reaktionsbereichs 34 durch Abpumpen von Sauerstoff O₂ mittels der Transfervorrichtung 10 zu regeln. Die Steuer- und Regeleinheit 32 ist hierfür zu einer Ansteuerung der Energiequelle 54 der Pumpeneinheit 12 vorgesehen. Die Pumprate der Transfervorrichtung 10 wird durch die angelegte Stromstärke beeinflusst. Ferner wird die Pumprate der Transfervorrichtung 10 durch eine Geometrie, insbesondere eine Oberfläche, des Zirkoniumoxidelements 20 beeinflusst. Während eines Betriebs wird bei einer gewählten Geometrie die Stromstärke der Energiequelle 54 variiert, um die geforderte Sauerstofftransferrate aus dem ersten Raum 14 in den zweiten Raum 18 einzustellen und den Sauerstoffpartialdruck in dem ersten Raum 14 auf einen vorgegebenen Wert zu regeln. Grundsätzlich kann die Pumpleistung auch durch weitere elektrische Betriebsbedingungen, wie beispielsweise über die Betriebstemperatur eingestellt werden. Die Pumprate der Transfervorrichtung 10 kann je nach Abmessungen des Zirkoniumoxidelements 20 und einer Höhe des Pumpenstroms im Bereich von wenigen ml pro Tag bis mehrere Liter pro Tag variiert werden.

Fig. 4 zeigt ein beispielhaftes Diagramm eines Meßprotokolls einer Gaskonzentration in dem ersten Raum 14, insbesondere ohne eine oben ausgeführte Regelung. Das Diagramm zeigt eine Sauerstoffkonzentration 62 sowie eine Kohlendioxidkonzentration 66 in dem Raum 14 in % über die Zeit t. Das Diagramm stellt dabei eine Variation einer Sauerstoff-Freisetzungsrate 58, 58' (dO₂/dt), jeweils zu einem Zeitpunkt t₁ und t₂ mittels einer Tangente dargestellt, aus der Photobioreaktoreinheit 36 in Abhängigkeit einer konstanten Kohlendioxid-Aufnahmerate 60, 60' (dCO₂/dt), jeweils zu einem Zeitpunkt t₁ und t₂ mittels einer Tangente dargestellt, in dem ersten Raum 14 dar. Bei konstanter Kohlendioxid-Aufnahmerate 60 nimmt die Sauerstoff-Freisetzungsrate 58 (dO₂/dt) mit zunehmender Sauerstoffkonzentration 62 im dem ersten Raum 14 ab. Nach dem ersten beispielhaften Spülvorgang 64 des ersten Raums 14 mit Stickstoff N₂ und der Zugabe von Kohlendioxid CO₂ ist die molare Sauerstoff-Freisetzungsrate 58 gleich der molaren Kohlendioxid-Aufnahmerate 60 (dCO₂/dt). Dieses Verhalten konnte bis zu einer Sauerstoffkonzentration 62 von ca. 15% in dem ersten Raum 14 beobachtet werden. Darüber nimmt die Sauerstoff-Freisetzungsrate 58 (dO₂/dt) deutlich ab. Es kann daher ein betragsmäßiges equimolares Verhältnis von dCO₂/dt = - dO₂/dt erreicht werden, wenn die Sauerstoffkonzentration 62 in dem ersten Raum 14 außerhalb des Reaktionsbereichs 34, aktiv auf einen reduzierten Wert von annähernd bis zu 15% gehalten wird. Daraus ergibt sich eine Regelgröße der Sauerstoffkonzentration 62 von annähernd 15%.

Bei einem Verfahren zum Betrieb der Anlage 16 wird mittels der Transfervorrichtung 10 ein absoluter Sauerstoffpartialanteil in dem ersten Raum 14 außerhalb eines Reaktionsbereichs 34 geregelt. Der absolute Sauerstoffpartialanteil in dem ersten Raum 14 außerhalb eines Reaktionsbereichs 34 wird mittels der Transfervorrichtung 10 auf einen Wert von annähernd 15% geregelt. Die Steuer- und Regeleinheit 32 steuert dazu die Transfervorrichtung 10 an. Die Steuer- und Regeleinheit 32 steuert dazu die Energiequelle 54 der Pumpeneinheit 12 der Transfervorrichtung 10 an. Durch die an der ersten Elektrode 50 und der zweiten Elektrode 52 anliegenden Spannung wird an der ersten Elektrode 50, welche als Kathode wirkt, Sauerstoff O₂ aus dem ersten Raum 14 durch Aufnahme von Elektronen zu Anionen O²⁻ reduziert. Diese Reaktion wird durch das als Katalysator dienende Platin der Elektrode 50 unterstützt. Anschließend wandern die die Anionen O²⁻ durch das Zirkoniumoxidelement 20, welches hierbei als lonenleiter und/oder Festkörperelektrolyt wirkt, zu der zweiten Elektrode 52. An der zweiten Elektrode 52 reagieren die Anionen O²⁻ in den zweiten Raum 18 wieder zu Sauerstoff O₂ durch Abgabe der Elektronen (Fig. 3).

Das Verfahren kann dabei grundsätzlich auch bei einer ISRU-Anwendung durchgeführt werden. Das Verfahren kann dabei zu der Abtrennung und/oder Anreicherung von Sauerstoff aus einem lokalen Gasgemisch und/oder aus lokalen Festkörpern im Rahmen von In-Situ Ressource Utilisation (ISRU) Anwendungen auf Planeten angewendet werden. Grundsätzlich kann die Anlage 16 dafür auch alternative Reaktoren umfassen. Insbesondere kann die Anlage 16 alternative Reaktoren für physiochemische, biochemische und/oder biologischen Prozesse, welche insbesondere Sauerstoff freisetzen, aufweisen.

Ferner kann das Verfahren grundsätzlich auch in einem Environmental Control System durchgeführt werden. Das Verfahren kann dabei zu einer Druck- und Temperaturregelung sowie zu einer Sauerstoffversorgung in einem zumindest im Wesentlichen geschlossenen System, wie insbesondere einem Raum-, Luft-, Land oder Wasserfahrzeug, verwendet werden. Grundsätzlich kann die Anlage 16 dafür auch alternative Reaktoren umfassen. Insbesondere kann die Anlage 16 alternative Reaktoren für physiko-chemische, biochemische und/oder biologische Prozesse aufweisen.

Ferner wird die Transfervorrichtung 10 zu einer Überwachung und zu einer Einstellung von sicherheitsrelevanten Betriebszuständen verwendet. Mittels der Messeinheit 26 der Transfervorrichtung 10 wird eine Spannungsdifferenz zwischen zwei Seiten 28, 30 der Pumpeneinheit 12 erfasst. Die Messeinheit 26 erfasst während eines Betriebs der Anlage 16 eine Spannungsdifferenz zwischen einer ersten, dem ersten Raum 14 zugewandten Seite 28 und einer zweiten, dem zweiten Raum 18 zugewandten Seite 30 der Pumpeneinheit 12. Des Weiteren schließt die Messeinheit 26 während eines Betriebs der Anlage 16, abhängig von der Spannungsdifferenz, auf eine Partialdruckdifferenz zwischen dem ersten Raum 14 und dem zweiten Raum 18 (Fig. 3).

Eine Durchführung des Verfahrens erfolgt unter Bedingungen reduzierter oder erhöhter Schwerkraft. Das Verfahren wird im Weltraum bei lokalen Schwerkraftswerten von 10⁻⁶ xg bis 10 xg durchgeführt. Das Verfahren wird insbesondere im Weltraum angewandt, wie beispielsweise bei µg in einem Raumschiff, einem Prozess in einem Raumschiff bei Beschleunigungen von 10⁻⁶ xg bis 10 xg, auf einem Planeten, wie dem Mars, und/oder auf einem Trabanten, wie dem Mond. Die Anlage 16 und/oder ein Reaktor der Anlage 16 können jedoch auch einer künstlichen Prozessbeschleunigung ausgesetzt sein, welche von den angegebenen Schwerkraftswerten abweicht. Die Schwerkraftswerte können so lokal beispielsweise verfahrenstechnisch drastisch erhöht werden, wie beispielsweise auf 100 xg.

## Patentansprüche

1. Anlage mit einem ersten abgeschlossenen Raum (14), einer Transfervorrichtung mit zumindest einer Pumpeneinheit (12), welche zu einem definierten Gastransfer zwischen dem ersten abgeschlossenen Raum (14) der Anlage (16) und einem von dem ersten Raum (14) getrennten zweiten Raum (18) vorgesehen ist, wobei die zumindest eine Pumpeneinheit (12) als eine selektive Sauerstoffpumpe ausgebildet ist **gekennzeichnet durch** eine Steuer- und/oder Regeleinheit (32), welche dazu vorgesehen ist, einen absoluten Sauerstoffpartialanteil in dem ersten Raum (14) außerhalb eines Reaktionsbereichs (34) mittels der Transfervorrichtung (10) zu steuern und/oder zu regeln, und zumindest eine Photobioreaktoreinheit (36), welche dazu vorgesehen ist, zugeführtes Kohlendioxid (CO₂) aufzunehmen und mittels Photosynthese Sauerstoff (O₂) zu erzeugen und abzugeben.

2. Anlage nach Anspruch 1,
**dadurch gekennzeichnet, dass**
die zumindest eine Pumpeneinheit (12) zumindest ein Zirkoniumoxidelement (20) aufweist, das zu einem selektiven Transfer von Sauerstoff (O₂) aus dem ersten Raum (14) in den zweiten Raum (18) vorgesehen ist.

3. Anlage nach Anspruch 2,
**dadurch gekennzeichnet, dass**
die zumindest eine Pumpeneinheit (12) zumindest ein Heizelement (22) aufweist, welches dazu vorgesehen ist, das zumindest eine Zirkoniumoxidelement (20) zu erhitzen.

4. Anlage nach Anspruch 2 oder 3,
**dadurch gekennzeichnet, dass**
das zumindest eine Zirkoniumoxidelement (20) der zumindest einen Pumpeneinheit (12) von einem dotierten Zirkoniumoxidelement gebildet ist.

5. Anlage nach einem der Ansprüche 2 bis 4,
**dadurch gekennzeichnet, dass**
das zumindest eine Zirkoniumoxidelement (20) der zumindest einen Pumpeneinheit (12) abhängig von einer Polarität (24) eines Pumpenstroms zu einem bidirektionalen Sauerstoff-Transfer zwischen dem ersten Raum (14) und dem zweiten Raum (18) vorgesehen ist.

6. Anlage nach einem der vorhergehenden Ansprüche,
**dadurch gekennzeichnet, dass**
die zumindest eine Pumpeneinheit (12) zu dem gezielten, selektiven Abtrennen von Sauerstoff (O₂) vorgesehen ist.

7. Anlage nach einem der vorhergehenden Ansprüche,
**dadurch gekennzeichnet, dass**
die zumindest eine Pumpeneinheit (12) in zumindest einem Betriebszustand zu einer elektrischen Reduktion zumindest eines Gases vorgesehen ist.

8. Anlage nach einem der vorhergehenden Ansprüche,
**dadurch gekennzeichnet, dass**
die zumindest eine Pumpeneinheit (12) zu einer Anreicherung eines reduzierten Gases in dem ersten Raum (14) und/oder dem zweiten Raum (18) vorgesehen ist.

9. Anlage nach einem der vorhergehenden Ansprüche,
**dadurch gekennzeichnet, dass**
die Transfervorrichtung (10) zumindest eine Messeinheit (26) aufweist, welche dazu vorgesehen ist, eine Spannungsdifferenz zwischen zumindest zwei Seiten (28, 30) der Pumpeneinheit (12) zu erfassen.

10. Anlage nach Anspruch 9,
**dadurch gekennzeichnet, dass**
die zumindest eine Messeinheit (26) dazu vorgesehen ist, abhängig von der Spannungsdifferenz auf eine Druckdifferenz zwischen dem ersten Raum (14) und dem zweiten Raum (18) zu schließen.

11. Verfahren zum Betrieb einer Anlage (16) nach einem der Ansprüche 1 bis 10.

12. Verfahren nach Anspruch 11,
**dadurch gekennzeichnet, dass**
mittels der Transfervorrichtung (10) ein absoluter Sauerstoffpartialanteil in dem ersten Raum (14) außerhalb eines Reaktionsbereichs (34) gesteuert und/oder geregelt wird.

13. Verfahren nach Anspruch 11 oder 12,
**dadurch gekennzeichnet, dass**
die Transfervorrichtung (10) zu einer Überwachung und/oder zu einer Einstellung zumindest eines sicherheitsrelevanten Betriebszustands verwendet wird.

14. Verfahren nach einem der Ansprüche 11 bis 13,
**dadurch gekennzeichnet, dass**
die Durchführung unter Bedingungen reduzierter oder erhöhter Schwerkraft erfolgt.

15. Verfahren nach einem der Ansprüche 11 bis 14,
**gekennzeichnet durch**
die Durchführung bei einer ISRU-Anwendung zu der Abtrennung und/oder Anreicherung von Sauerstoff aus einem Gasgemisch und/oder aus lokal vorliegendem Material im Rahmen der In-Situ Ressource Utilisation (ISRU) Anwendung auf Planeten.

16. Verfahren zumindest nach einem der Ansprüche 11 bis 14,
**gekennzeichnet durch**
die Durchführung in einem Environmental Control System, und zwar in einem Klimasystem, welches zu einem Luftaustausch, einer Druckregelung, einer Temperaturregelung, einer Druckversorgung und/oder einer Sauerstoffversorgung in einer zumindest im Wesentlichen geschlossenen Kabine vorgesehen ist.

## Claims

1. Installation with a first closed space (14), with a transfer device comprising at least one pump unit (12) that is configured for a defined gas transfer between a first, closed-off space (14) of the installation (16) and a second space (18) which is separate from the first space (14), the pump unit (12) being realised as a selective oxygen pump, **characterised by** a control and/or regulation unit (32), which is configured to control and/or regulate via the transfer device (10) an absolute partial fraction of oxygen in the first space (14) outside a reaction zone (34), and by at least one photobioreactor unit (36), which is configured to receive fed-in carbon dioxide (CO₂) and to generate oxygen (O₂) via photosynthesis and to discharge the latter.

2. Installation according to claim 1, **characterised in that** the at least one pump unit (12) comprises at least one zirconium oxide element (20), which is configured for a selective transfer of oxygen (O₂) out of the first space (14) into the second space (18).

3. Installation according to claim 2, **characterised in that** the at least one pump unit (12) comprises at least one heating element (22), which is configured for heating the at least one zirconium oxide element (20).

4. Installation according to claim 2 or 3, **characterised in that** the at least one zirconium oxide element (20) of the at least one pump unit (12) is implemented by a doped zirconium oxide element.

5. Installation according to one of claims 2 to 4, **characterised in that** the at least one zirconium oxide element (20) of the at least one pump unit (12) is configured, depending on a polarity (24) of a pump flow, for a bi-directional oxygen transfer between the first space (14) and the second space (18).

6. Installation according to one of the preceding claims, **characterised in that** the at least one pump unit (12) is configured for specifically and selectively separating off oxygen (O₂).

7. Installation according to one of the preceding claims, **characterised in that** the at least one pump unit (12) is in at least one operating state configured for an electrical reduction of at least one gas.

8. Installation according to one of the preceding claims, **characterised in that** the at least one pump unit (12) is configured for an accumulation of a reduced gas in the first space (14) and/or in the second space (18).

9. Installation according to one of the preceding claims, **characterised in that** the transfer device (10) comprises at least one measuring unit (26), which is configured for capturing a voltage difference between at least two sides (28, 30) of the pump unit (12).

10. Installation according to claim 9, **characterised in that** the at least one measuring unit (26) is configured for deducting, on the basis of the voltage difference, a pressure difference between the first space (14) and the second space (18).

11. Method for operating an installation (16) according to one of claims 1 to 10.

12. Method according to claim 11, **characterised in that** an absolute oxygen partial fraction in the first space (14) outside a reaction area (34) is controlled and/or regulated via the transfer device (10).

13. Method according to claim 11 or 12, **characterised in that** the transfer device (10) is used for a monitoring and/or for an adjustment of at least one safety-relevant operating state.

14. Method according to one of claims 11 to 13, **characterised in that** the implementation is realised under conditions of reduced or increased gravity.

15. Method according to one of claims 11 to 14, **characterised by** the implementation in an ISRU application for a separating-off and/or accumulation of oxygen from a gas mixture and/or from a locally available material in a context of an In Situ Resource Utilisation (ISRU) on planets.

16. Method at least according to one of claims 11 to 14, **characterised by** the implementation in an Environmental Control System, namely in a climate system that is configured for an air exchange, a pressure regulation, a temperature regulation, a pressure supply and/or an oxygen supply in an at least substantially closed-off cabin.

## Revendications

1. Installation avec un premier espace fermé (14), avec un dispositif de transfert comprenant au moins une unité de pompe (12) configurée pour un transfert défini de gaz entre le premier espace fermé (14) de l'installation (16) et un deuxième espace (18), qui est séparé du premier espace (14), l'au moins une unité de pompe (12) étant réalisée comme pompe d'oxygène sélective,
**caractérisé par** une unité de commande et/ou régulation (32) configurée pour une commande et/ou régulation d'une fraction partielle d'oxygène absolue dans le premier espace (14) au dehors d'une zone de réaction (34) par le biais du dispositif de transfert (10), et par au moins une unité photobioréacteur (36) configurée pour accueillir de dioxyde de carbone alimenté (CO₂) et pour générer d'oxygène (O₂) par photosynthèse et l'émettre.

2. Installation selon la revendication 1,
**caractérisé en ce que** l'au moins une unité de pompe (12) comporte au moins un élément d'oxyde de zirconium (20), configuré pour un transfert sélectif d'oxygène (O₂) du premier espace (14) dans le deuxième espace (18).

3. Installation selon la revendication 2,
**caractérisé en ce que** l'au moins une unité de pompe (12) comporte au moins un élément de chauffage (22), configuré pour chauffer l'au moins un élément d'oxyde de zirconium (20).

4. Installation selon l'une quelconque des revendications 2 ou 3,
**caractérisé en ce que** l'au moins un élément d'oxyde de zirconium (20) de l'au moins une unité de pompe (12) est implémenté par un élément d'oxyde de zirconium dopé.

5. Installation selon l'une quelconque des revendications 2 à 4,
**caractérisé en ce qu',** en dépendance à une polarité (24) d'un flux de pompe, l'au moins un élément d'oxyde de zirconium (20) de l'au moins une unité de pompe (12) est configuré pour un transfert d'oxygène bidirectionnel entre le premier espace (14) et le deuxième espace (18).

6. Installation selon l'une quelconque des revendications précédentes,
**caractérisé en ce que** l'au moins une unité de pompe (12) est configurée pour une séparation d'oxygène (O₂) ciblée et sélective.

7. Installation selon l'une quelconque des revendications précédentes,
**caractérisé en ce qu'**en au moins un état d'opération l'au moins une unité de pompe (12) est configurée pour une réduction électrique d'au moins un gaz.

8. Installation selon l'une quelconque des revendications précédentes,
**caractérisé en ce que** l'au moins une unité de pompe (12) est configurée pour une accumulation d'un gaz réduit dans le premier espace (14) et/ou dans le deuxième espace (18).

9. Installation selon l'une quelconque des revendications précédentes,
**caractérisé en ce que** le dispositif de transfert (10) comporte au moins une unité de mesurage (26) configurée pour capturer une différence de voltage entre au moins deux côtés (28, 30) de l'unité de pompe (12).

10. Installation selon la revendication 9,
**caractérisé en ce que** l'au moins une unité de mesurage (26) est configurée pour déduire, en dépendance à la différence de voltage, une différence de pression entre le premier espace (14) et le deuxième espace (18).

11. Procédé en fonctionnement d'une installation (16) selon l'une quelconque des revendications 1 à 10.

12. Procédé selon la revendication 11,
**caractérisé en ce qu'**une fraction partielle d'oxygène absolue dans le premier espace (14) au dehors d'une zone de réaction (34) est commandée et/ou régulée par le biais du dispositif de transfert (10).

13. Procédé selon la revendication 11 ou 12,
**caractérisé en ce que** le dispositif de transfert (10) est usé pour un monitorage et/ou pour un ajustement d'au moins un état d'opération relevant quant à la sécurité.

14. Procédé selon l'une quelconque des revendications 11 à 13,
**caractérisé en ce que** la mise en oeuvre est effectuée sous conditions de gravité réduite ou augmentée.

15. Procédé selon l'une quelconque des revendications 11 à 14,
**caractérisé par** la mise en oeuvre dans une application ISRU (utilisation de ressources in situ) pour une séparation et/ou accumulation d'oxygène d'un mélange gazeux et/ou d'un matériau présent localement, dans le cadre d'une utilisation de ressources in situ (ISRU) appliquée sur des planètes.

16. Procédé au moins selon l'une quelconque des revendications 11 à 14,
**caractérisé par** la mise en oeuvre dans un système de contrôle environnemental (Environmental Control System), notamment dans un système climatique configuré pour un échange d'air, une régulation de pression, une régulation de température, un approvisionnement de pression et/ou un approvisionnement d'oxygène dans une cabine au moins sensiblement fermée.
